# EUROPEAN PATENT APPLICATION

(11) **EP 4 843 565 A1**
(43) Date of publication of application: **25.11.2026**
(21) Application number: 25177730.6
(22) Date of filing: 20.05.2025
(51) Int. Cl.: A61K 9/20, A61K 31/519

(54) **IMMEDIATE RELEASE PHARMACEUTICAL TABLET FORMULATION CONTAINING RUXOLITINIB HEMIFUMARATE**

(71) Applicant: Adalvo Limited, San Gwann, SGN 3000 (MT)
(72) Inventor: Bayrak, Gokhan, Istanbul (TR)
(74) Representative: Harber IP s.r.o.

(57) **Abstract**

The invention relates to a solid oral immediate-release pharmaceutical tablet formulation comprising a therapeutically effective amount of Ruxolitinib hemifumarate and one or more pharmaceutically acceptable excipients, wherein the formulation is free of lactose, and is obtained by solvent- free tablet manufacturing method.

## Description

### FIELD OF THE INVENTION

The present invention relates to a stable immediate release pharmaceutical tablet formulation containing Ruxolitinib hemifumarate prepared by solvent-free pharmaceutical tablet manufacturing method.

### BACKGROUND OF THE INVENTION

Ruxolitinib (INCB018424 phosphate, INC424, ruxolitinib phosphate) is a selective inhibitor of Janus Associated Kinase 1 (JAK1) and JAK2 (IC50 values of 3.3 nM and 2.8 nM, respectively) which mediates the signalling of a number of cytokines and growth factors that are important for haematopoiesis and immune function. The chemical name is (R)-3-(4-(7H-pyrrolo [2, 3-d] pyrimidin-4-yl)-1H-pyrazol-1-yl)-3-cyclopentylpropanenitrile phosphate. The molecular formula is C17H21N6O4P and the molecular weight is 404.36.

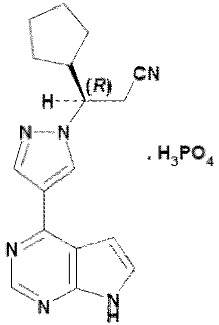

Ruxolitinib phosphate is a white to almost white, non-hygroscopic powder, highly soluble in water, and the solubility is pH dependent.

Tablets containing ruxolitinib phosphate have been approved in the US and Europe for the treatment of myelofibrosis, polycythemia vera and graft-versus-host disease. These tablets, marketed as Jakavi^{®} in Europe and as Jakafi^{®} in the US, are immediate-release tablets containing 5 mg, 15 mg and 20 mg of ruxolitinib (as ruxolitinib phosphate) as active substance. Other ingredients are lactose monohydrate, microcrystalline cellulose, magnesium stearate, colloidal anhydrous silica, sodium starch glycolate, povidone, hydroxypropyl cellulose. The tablet manufacturing process is based upon wet granulation.

Different salts of Ruxolitinib are described in the prior art, like phosphate, malate, sulphate (WO2008157208), hydrobromic acid, hydrochloric acid, citric acid, fumaric acid, L-tartaric acid, p-toluenesulphonic acid, benzoic acid, benzenesulphonic acid, ethanesulphonic acid, 2-naphthalenesulphonic acid and 4- chlorobenzenesulphonic acid salts (WO2016074650A). Ruxolitinib hemifumarate was proposed as alternative salt to phosphate, and crystal modification 1 thereof is described in WO2017125097.

Pharmaceutical formulations containing ruxolitinib hemifumarate are known from WO2024028193A1. The application describes solid oral immediate-release pharmaceutical formulations comprising a therapeutically effective amount of Ruxolitinib hemifumarate, and provides examples wherein the immediate release tablets include lactose monohydrate, microcrystalline cellulose, magnesium stearate, colloidal anhydrous silica, sodium starch glycolate, povidone, hydroxypropyl cellulose. Tablets were prepared by wet granulation.

Stability testing was conducted on the tablets described in Example 2 under accelerated conditions of 40 ± 2°C and 75 ± 5% relative humidity to assess their long-term stability:

| | t = 0 | 1 month (40±2°C, 75±5% RH) | 2 months (40±2°C, 75±5% RH) | 3 months (40±2°C, 75±5% RH |
|---|---|---|---|---|
| Maximum individual impurity | 0.00% | 0.02% | 0.03% | 0.04% |
| Total | 0.00% | 0.02% | 0.03% | 0.04% |

The prior art describes tablet formulations containing Ruxolitinib phosphate and hemifumarate produced via wet granulation, a process that involves multiple steps including binder addition, wet massing, drying, and milling, leading to increased complexity, longer processing times, and potential stability challenges. This underscores the need for a more efficient and robust manufacturing alternative and for tablets, particularly to address stability concerns related to hydrolytic impurities. Current formulations may be susceptible to moisture-induced degradation, leading to reduced shelf life and compromised efficacy.

Furthermore, the prior art formulations contain lactose as an excipient, creating a need for lactose-free alternatives to accommodate lactose-intolerant patients.

### SUMMARY OF THE INVENTION

The present invention addresses the above drawbacks by utilizing solvent-free tablet manufacturing methods, like direct compression or dry granulation, for preparation of a pharmaceutical tablet formulation containing Ruxolitinib hemifumarate, which eliminates the need for liquid binders and drying steps, thereby streamlining production, reducing energy consumption, and minimizing degradation risks. Additionally, solvent-free tablet manufacturing methods enhance tablet stability by avoiding exposure to moisture, making it particularly advantageous for hygroscopic or heat-sensitive active ingredients. By simplifying the manufacturing process while improving product reliability and stability, the invention offers a more scalable and cost-effective solution compared to conventional wet granulation methods. Moreover, the inventive formulation has improved dissolution properties.

Further, unlike the prior art formulations that contain lactose, the tablet formulation according to the invention is lactose-free, which is beneficial for lactose-intolerant individuals, improving patient compliance and safety.

### DETAILED DESCRIPTION OF THE INVENTION

The invention provides a stable solid oral immediate-release pharmaceutical tablet formulation containing a therapeutically effective amount of Ruxolitinib hemifumarate, and one or more pharmaceutically acceptable excipient, avoiding lactose and obtained by solvent-free tablet manufacturing method.

A "therapeutically active amount " refers to the minimum quantity of the active pharmaceutical ingredient Ruxolitinib required to produce a desired medicinal effect in a patient, while maintaining an acceptable safety profile.

Ruxolitinib hemifumarate used for preparation of the stable solid oral immediate-release pharmaceutical tablet formulation can be in crystalline or amorphous form, or a mixture thereof. In a preferred embodiment Ruxolitinib hemifumarate is in crystalline Form 1, showing an x-ray powder diffraction pattern characteristic peaks at about 5.6, 13.1, 18.1, 22.6, 23.5 and 25.5 +/- 0.2_{°} 2-theta measured by CuKalpha radiation.

"Pharmaceutically acceptable excipients" refer to inactive, non-therapeutic substances intentionally added to a drug formulation to aid in manufacturing, stability, delivery, or patient acceptability.

The pharmaceutical formulation according to the present invention is in form of an immediate-release tablet, comprising Ruxolitinib hemifumarate and one or more excipients selected from the group of diluent, disintegrant, at least one binder, glidant and lubricant.

A diluent is a pharmaceutically acceptable excipient primarily used to increase the bulk volume of a solid dosage form, such as tablets or capsules, ensuring uniform and accurate dosing of the active pharmaceutical ingredient. Examples of diluents are Microcrystalline Cellulose, silicified Microcrystalline Cellulose, Starch (corn, potato, pregelatinized starch), Powdered Cellulose, Mannitol, Sorbitol, Dicalcium Phosphate, Calcium Carbonate, Calcium Sulfate. The preferred diluent is Microcrystalline Cellulose.

A disintegrant is a pharmaceutically acceptable excipient added to solid dosage forms to promote rapid breakup or disintegration when exposed to fluids (e.g., water, gastric juice). This ensures the release and dissolution of the active pharmaceutical ingredient for optimal absorption in the gastrointestinal tract. Examples of disintegrants are Sodium Starch Glycolate, Croscarmellose Sodium, Crospovidone, Low-Substituted Hydroxypropyl Cellulose, Pregelatinized Starch, Alginic Acid, and Sodium Alginate. The preferred disintegrant is Sodium Starch Glycolate.

A binder is a pharmaceutically acceptable excipient used in direct compression tablet formulations to improve powder cohesion and ensure the tablet maintains its mechanical strength after compaction. Examples of binders are Povidone, Hydroxypropyl Cellulose, Hydroxypropyl Methylcellulose, Polyvinyl Alcohol, Pregelatinized Starch (such as Partially Pregelatinized Maize Starch), Sodium Alginate, Microcrystalline Cellulose (MCC) + Silica (e.g., Prosolv^{®}). The preferred binder is Povidone.

A glidant and lubricant are pharmaceutically acceptable excipients added to powder blends in direct compression to improve powder flowability by reducing interparticle friction and cohesion. This ensures uniform die filling during tablet compression, minimizing weight variation and enhancing production efficiency. Glidants improve powder flow (pre-compression), Lubricants reduce ejection forces (post-compression). Examples of glidants are Colloidal Silicon Dioxide, Calcium Phosphate Tribasic, Magnesium Stearate, and Talc. Examples of lubricants are Magnesium Stearate, Calcium Stearate, Stearic Acid, Sodium Stearyl Fumarate, and Talc. The preferred glidant is Colloidal Silicon Dioxide, the preferred lubricant is Magnesium Stearate.

In a preferred embodiment of the invention the solid oral immediate-release tablet comprises from 3 % w/w to 6 % w/w of Ruxolitinib hemifumarate,
from 85 to 90 % w/w of a diluent,
from 2 to 6 % w/w of a disintegrant,
from 2 to 6 % w/w of a binder,
from 0.5 to 2 % w/w of a glidant, and
from 0.5 to 2 % w/w of a lubricant,
calculated based on the total weight of the tablet.

According to the invention the solid oral immediate-release tablet preferably comprises Ruxolitinib hemifumarate, a diluent Microcrystalline Cellulose, a disintegrant Sodium Starch Glycolate, a binder Povidone and/or Hydroxypropyl cellulose, a glidant Colloidal Silicon Dioxide and a lubricant Magnesium Stearate.

In a preferred embodiment of the invention the solid oral immediate-release tablet comprises
3.7 % w/w of Ruxolitinib hemifumarate
87.8 % w/w of Microcrystalline Cellulose
3 % w/w of Sodium Starch Glycolate
4 % w/w of Povidone
1 % w/w of Colloidal Silicon Dioxide and
0.5 % w/w of Magnesium Stearate.

In a more preferred embodiment of the invention the solid oral immediate-release tablet comprises:
3.7 % w/w of Ruxolitinib hemifumarate
87.8 % w/w of Microcrystalline Cellulose
3 % w/w of Sodium Starch Glycolate
2 % w/w of Povidone
2 % w/w of Hydroxypropyl cellulose
1 % w/w of Colloidal Silicon Dioxide and
0.5 % w/w of Magnesium Stearate.

The solid oral immediate-release tablet comprising Ruxolitinib hemifumarate and one or more excipients selected from the group of diluent, disintegrant, at least one binder, glidant and lubricant is prepared by solvent free tablet manufacturing methods including dry granulation (slugging/roller compaction) and direct compression.

Dry granulation is a tablet manufacturing method used when APIs are sensitive to moisture or heat, eliminating the need for liquid binders. The process involves two main techniques: slugging and roller compaction.

Steps in dry granulation:
- Blending
   - - The API and excipients (diluent, disintegrant, binder, lubricants, glidant) are mixed to ensure uniformity.
- Compaction
   - - Slugging: The powder blend is compressed into large, flat tablets (slugs) using a tablet press.
   - - Roller Compaction: The powder is passed between counter-rotating rollers to form a compacted ribbon or flakes.
- Milling/Sieving
   - - The slugs or ribbons are crushed and sieved to produce uniform granules.
- Final Blending
   - - Additional excipients (disintegrant, lubricant) are mixed with the granules.
- Tableting
   - - The granules are compressed into tablets.

Direct compression is the simplest and most efficient tablet manufacturing method, where powdered API is mixed with suitable excipients and compressed directly into tablets without intermediate granulation steps.

Steps in direct compression:
- Sifting
   - - The API and direct compression-friendly excipients such as a diluent, a disintegrant, at least one binder, a lubricant, and a glidant are sieved to ensure uniform particle size and prevent segregation.
- Blending
   - - The API is mixed with direct compression-friendly excipients such as a diluent, a disintegrant, at least one binder, a lubricant, and a glidant.
- Compression
   - - The homogeneous blend is fed into a tablet press and compressed into tablets of the desired shape, size, and hardness.

According to the present invention the solid oral immediate-release tablet is prepared by dry granutation of Ruxolitinib hemifumarate and the one or more pharmaceutically acceptable excipients selected from a diluent, a disintegrant, at least one binder, a glidant and a lubricant, to form a granulate; a step of combining the granulate with a disintegrant and a lubricant to form a mixture; and a step of direct compaction of the mixture; wherein all steps are performed solvent-free; and wherein the dry granulation preferably includes at least one of slugging, compaction and roller compaction.

According to the present invention the solid oral immediate-release tablet is prepared by direct compression comprising a step of combining Ruxolitinib hemifumarate and the one or more pharmaceutically acceptable excipients selected from a diluent, a disintegrant, at least one binder, a glidant and a lubricant, to form a mixture; and a step of direct compaction of the mixture; wherein all steps are performed solvent-free.

### BRIEF DESCRIPTION OF THE DRAWING

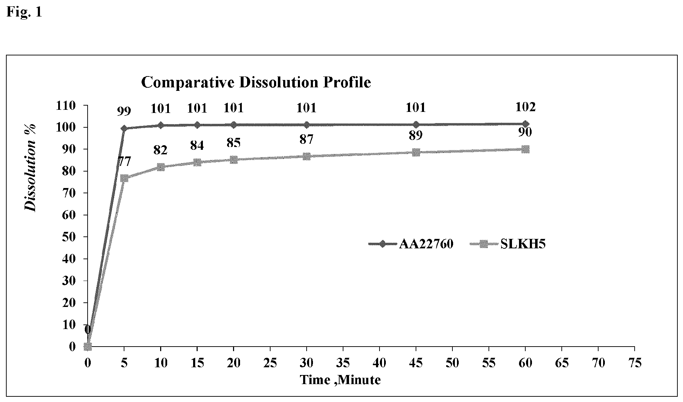

Fig. 1: Dissolution profile of the tablets according to the present invention example 2.1 compared with the dissolution profile of the commercial tablets Jakavi(R) in 500ml of 0.1N HCl at 37_{°}C±0.5_{°}C using USP apparatus 2 (paddle) at 50 rpm. Test batch (Lot No: AA22760) and reference batch (Lot No: SLKH5).

### EXAMPLES

### Stability testing

Tablets packed in OPA/ALU/PVC (ALU ≥7µm) were tested at 40°C ± 2°C and 75% ± 5% relative humidity (RH).

The API is susceptible to the formation of hydrolytic impurities under typical manufacturing and storage conditions. Known Ruxolitinib hydrolytic impurities:

### Acid impurity:

3-(4-(7H-pyrrolo[2, 3-d]pyrimidin-4-yl)-1H-pyrazol-1-yl)-3-cyclopentyl propanoic acid, and Amid impurity:
3-(4-(7H-Pyrrolo[2,3-d]pyrimidin-4-yl)-1H-pyrazol-1-yl)-3-cyclopentyl propanamide

### Impurity analysis method

Related substances were analyzed by high-performance liquid chromatography (HPLC) Chromatographic Conditions
Column: GL Sciences Inertsil ODS-3, 4.6 mm × 250 mm, 5µm or equivalent
Detector: UV-PDA
Wavelength: 250 nm
Column Temperature: 35°C
Sample temperature: 5°C
Flow Rate: 0.9 ml/min.
Injection Volume: 60 µl
Elution : Gradient
Run Time: 75 minutes
Diluent: Methanol : 0.1% Phosphoric acid (2:8), v/v
Mobile Phase A: pH 5.5 Ammonium Acetate Buffer
Mobile Phase B: Acetonitrile
Needle Wash: Methanol : Purified water (50:50), % v/v
Note-1: Needle washing parameters for Shimadzu device are "Rinsing Volume=2000µl" and "Dip Time=10 seconds

### Gradient Program

| **Time (minutes)** | **Mobile Phase A%** | **Mobile Phase B%** |
|---|---|---|
| 0 | 90 | 10 |
| 10 | 70 | 30 |
| 35 | 65 | 35 |
| 50 | 30 | 70 |
| 65 | 30 | 70 |
| 67 | 90 | 10 |
| 75 | 90 | 10 |

### Preparation of Solutions

### Preparation of pH 5.5 Ammonium Acetate Buffer Solution:

Weigh 0.77 g of Ammonium acetate and transfer into the glass bottle. Dissolve with 950 ml deionized water. Adjust pH to 5.5 ± 0.05 with diluted acetic acid. Make up to 1000 ml with deionized water, filter with a 0.45 µm nylon membrane filter and then degas.

### Preparation of 0.1% Phosphoric acid solution:

Dissolve 1 ml of Ortho-phosphoric acid 85% in 1000 ml of deionized water. Shake well.

### Preparation of mobile phase A:

Use pH 5.5 Ammonium Acetate Buffer as mobile phase A.

### Preparation of mobile phase B

Use Acetonitrile as mobile phase B.

### Chromatographic Procedure:

1. Inject blank solution once
2. Inject placebo and placebo containing active solution once
3. Inject Standard Solution six times
   - %RSD value for six standard injections should be no more than 5.0%
   - In the chromatogram obtained with the standard solution, the symmetry factor of the Ruxolitinib peak is between 0.8-1.8.
   - In the chromatogram obtained with the standard solution, the theoretical plate number of the Ruxolitinib peak should be min. 2000.
4. Inject sample solutions once.
5. The standart solution is injected into the system once at the end of all injections.
   - The cumulative %RSD value for 6 standard injections together with the Final Control standard should be more than 10.0%.

### Dissolution testing

### Dissolution Parameters

Medium: 0.1 N HCl
Volume: 500 ml
Apparatus: Paddle (Apparatus II)
Speed: 50 rpm
Time: 15 minutes
Temperature: 37°C ± 0.5°C
Sampling Volume: 5 ml
Diluent: 0.1 N HCl

### Example 1. Immediate release tablet prepared by direct compression

| **Raw Material** | **Unit Formula (mg/tablet)** | **Ratio % (w/w)** | **Function** |
|---|---|---|---|
| Ruxolitinib Hemifumarate¹ | 23.79 | 3.72 | API |
| Microcrystalline Cellulose Type 102 | 561.81 | 87.78 | Diluent |
| Sodium Starch Glycolate (Type A) | 19.20 | 3.00 | Disintegrant |
| Povidone K30 | 25.60 | 4.00 | Binder |
| Colloidal Silicon Dioxide | 6.40 | 1.00 | Glidant |
| Magnesium Stearate | 3.20 | 0.50 | Lubricant |
| **Tablet Total Weight** | **640.00** | 100 | - |

| | | | |
|---|---|---|---|
| ¹ Equivalent to 20 mg ruxolitinib base. | | | |

### Manufacturing Process:

1. Ruxolitinib Hemifumarate, Microcrystalline Cellulose Type 102, Sodium Starch Glycolate (Type A), Povidone K30, Colloidal Silicon Dioxide, Magnesium Stearate are dispensed.
2. Ruxolitinib Hemifumarate, Microcrystalline Cellulose Type 102, Sodium Starch Glycolate (Type A), Povidone K30, Colloidal Silicon Dioxide are transferred to the Suitable mixing system and mixed.
3. The powder is sifted through 0.8mm sieve.
4. The powder is transferred to the Suitable mixing system and mixed.
5. Magnesium Stearate is sifted through 0.60mm sieve.
6. Sifted Magnesium Stearate is added to the Suitable mixing system and mixed.
7. Tablet compression is carried out with the prepared final powder under appropriate parameters.

### Example 2. Immediate release tablets prepared by direct compression

| **Raw Materials** | **Function** | **20 mg (mg/tablet)** | **15 mg (mg/tablet)** | **10mg (mg/tablet)** | **5mg (mg/tablet)** |
|---|---|---|---|---|---|
| | | **Example 2.1** | **Example 2.2** | **Example 2.3** | **Example 2.4** |
| Ruxolitinib Hemifumarate | API | 23.79 | 17.84 | 11.89 | 5.95 |
| Microcrystalline Cellulose Type 102 | Diluent | 561.81 | 421.36 | 280.91 | 140.45 |
| Sodium Starch Glycolate (Type A) | Disintegrant | 19.20 | 14.40 | 9.60 | 4.80 |
| Hydroxypropyl Cellulose (EXF) | Binder | 12.80 | 9.60 | 6.40 | 3.20 |
| Povidone K30 | Binder | 12.80 | 9.60 | 6.40 | 3.20 |
| Colloidal Silicon Dioxide | Glidant | 6.40 | 4.80 | 3.20 | 1.60 |
| Magnessium Stearate | Lubricant | 3.20 | 2.40 | 1.60 | 0.80 |
| **Tablet weight** | | 640.00 | 480.00 | 320.00 | 160.00 |

### Manufacturing Process:

**1.** Ruxolitinib Hemifumarate, Microcrystalline Cellulose Type 102, Sodium Starch Glycolate (Type A), Hydroxypropyl Cellulose (EXF), Povidone K30, Colloidal Silicon Dioxide, Magnesium Stearate are dispensed.
**2.** Ruxolitinib Hemifumarate, Microcrystalline Cellulose Type 102, Sodium Starch Glycolate (Type A), Hydroxypropyl Cellulose (EXF), Povidone K30, Colloidal Silicon Dioxide are transferred to the mixer and mixed.
**3.** The blend is sifted through 0.8mm sieve.
**4.** The blend is transferred to the mixer and mixed.
**5.** Magnesium Stearate is sifted through 0.60mm sieve.
**6.** Sifted Magnesium Stearate is added to the mixer and mixed.
**7.** Tablet compression is carried out with the prepared final blend using appropriate parameters.

### Stability data of the tablets prepared according to Example 2.1

Tablets prepared according to Example 2.1 were packed in OPA/ALU/PVC (ALU ≥7µm) and tested at 40°C ± 2°C and 75% ± 5% relative humidity RH.

| | T=0 | 3 months |
|---|---|---|
| Maximum individual impurity | 0.022 % | 0.021 % |
| Acid impurity | ND | ND |
| Amide impurity | ND | ND |

| | | |
|---|---|---|
| ND - not detected | | |

### Example 3. Immediate release tablets prepared by using compaction

| **Raw Material** | **Unit Formula (mg/tablet)** | **Ratio % (w/w)** | **Function** |
|---|---|---|---|
| Ruxolitinib Hemifumarate¹ | 23,79 | 3,72 | API |
| Microcrystalline Cellulose Type 102 | 561,81 | 87,78 | Diluent |
| Sodium Starch Glycolate (Type A) | 19,20 | 3,00 | Disintegrant |
| Povidone K30 | 25,60 | 4,00 | Binder |
| Colloidal Silicon Dioxide | 6,40 | 1,00 | Glidant |
| Magnesium Stearate | 3,20 | 0,50 | Lubricant |
| **Tablet Total Weight** | **640,00** | 100 | - |

| | | | |
|---|---|---|---|
| ¹ Equivalent to 20 mg ruxolitinib base. | | | |

### Manufacturing Process:

1. Ruxolitinib Hemifumarate, Microcrystalline Cellulose Type 102, Sodium Starch Glycolate (Type A), Povidone K30, Colloidal Silicon Dioxide, Magnesium Stearate are dispensed.
2. Ruxolitinib Hemifumarate, Microcrystalline Cellulose Type 102, half amount of the Sodium Starch Glycolate (Type A), Povidone K30, Colloidal Silicon Dioxide are transferred to the suitable mixing system and mixed.
3. The powder is sifted through 0.8mm sieve.
4. The powder is lubricated with half amount of the Magnesium Stearate sifted through 0.6 mm sieve
5. Above obtained blend compressed in tablet compression machine with suitable parameters and sieved through suitable sieving system (e.g. oscillating sieve) and sieve size (e.g. 1.0 mm).
6. Half amount of the Sodium Starch Glycolate (Type A) is added to the above blend.
7. The powder is transferred to the Suitable mixing system and mixed.
8. The above mixture is lubricated with half amount of the Magnesium Stearate that sifted through 0.60mm sieve.
9. Tablet compression is carried out with the prepared final powder under appropriate parameters.

### Example 4. Immediate release tablets prepared by using compaction

| **Raw Material** | **Unit Formula (mg/tablet)** | **Ratio % (w/w)** | **Function** |
|---|---|---|---|
| Ruxolitinib Hemifumarate¹ | 23,79 | 3,72 | API |
| Microcrystalline Cellulose Type 102 | 561,81 | 87,78 | Diluent |
| Sodium Starch Glycolate (Type A) | 19,20 | 3,00 | Disintegrant |
| Povidone K30 | 25,60 | 4,00 | Binder |
| Colloidal Silicon Dioxide | 6,40 | 1,00 | Glidant |
| Magnesium Stearate | 3,20 | 0,50 | Lubricant |
| **Tablet Total Weight** | **640,00** | 100 | - |

| | | | |
|---|---|---|---|
| ¹ Equivalent to 20 mg ruxolitinib base. | | | |

### Manufacturing Process:

1. Ruxolitinib Hemifumarate, Microcrystalline Cellulose Type 102, Sodium Starch Glycolate (Type A), Povidone K30, Colloidal Silicon Dioxide, Magnesium Stearate are dispensed.
2. Ruxolitinib Hemifumarate, Microcrystalline Cellulose Type 102, half amount of the Sodium Starch Glycolate (Type A), Povidone K30, Colloidal Silicon Dioxide are transferred to the suitable mixing system and mixed.
3. The powder is sifted through 0.8mm sieve.
4. The powder is lubricated with half amount of the Magnesium Stearate sifted through 0.6 mm sieve
5. Above obtained blend compacted with suitable parameters and sieved through suitable sieving system (e.g. oscillating sieve) and sieve size (e.g. 1.0 mm).
6. Half amount of the Sodium Starch Glycolate (Type A) is added to the above blend.
7. The powder is transferred to the Suitable mixing system and mixed.
8. The above mixture is lubricated with half amount of the Magnesium Stearate that sifted through 0.60mm sieve.
9. Tablet compression is carried out with the prepared final powder under appropriate parameters.

### Example 5.

Bioequivalence of the test product described in example 2.1 was demonstrated in an open-label, randomized, balanced, two-treatment, two-period, two-sequence, single-dose, crossover study conducted in healthy adult human subjects under fasting conditions. The trial compared the pharmacokinetic profiles of the test and reference (Jakavi^{®} 20 mg) formulations, with participants receiving both products in randomized order, separated by an appropriate washout period.

| **PARAMETER** | **REFERENCE** | **TEST** | **ISCV (%)** | **RATIO (%)** | **90% CI** |
|---|---|---|---|---|---|
| Cmax (ng/mL) | 490.907 | 478.641 | 12.933 | 97.50 | 93.25-101.95 |
| AUCt (ng/mL)* (hr) | 2167.913 | 2107.717 | 12.708 | 97.22 | 93.05-101.58 |

## Claims

1. A solid oral immediate-release pharmaceutical tablet formulation comprising a therapeutically effective amount of Ruxolitinib hemifumarate and one or more pharmaceutically acceptable excipients, wherein the formulation is free of lactose, and is obtained by solvent-free tablet manufacturing method.

2. The solid oral immediate-release pharmaceutical tablet formulation according to claim 1 wherein Ruxolitinib hemifumarate is in crystalline form, preferably in crystalline Form 1 showing an x-ray powder diffraction pattern characteristic peaks at about 5.6, 13.1, 18.1, 22.6, 23.5 and 25.5 +/- 0.2∘ 2-theta measured by CuKalpha radiation.

3. The solid oral immediate-release pharmaceutical tablet formulation according to claim 1 or 2, wherein the one or more pharmaceutically acceptable excipients are selected from the group of diluent, disintegrant, at least one binder, glidant and lubricant.

4. The solid oral immediate-release pharmaceutical tablet formulation according to any one of claims 1 to 3, wherein the diluent is selected as one from the group of Microcrystalline cellulose, silicified Microcrystalline cellulose, Corn Starch, Potato Starch, Pregelatinized Starch, Powdered Cellulose, Mannitol, Sorbitol, Dicalcium Phosphate, Calcium Carbonate, Calcium Sulfate.

5. The solid oral immediate-release pharmaceutical tablet formulation according to claim 4, wherein the diluent is Microcrystalline cellulose.

6. The solid oral immediate-release pharmaceutical tablet formulation according to any one of claims 1 to 5, wherein the disintegrant is selected from Sodium starch glycolate, Croscarmellose Sodium, Crospovidone, Low-Substituted Hydroxypropyl Cellulose, Pregelatinized Starch, Alginic Acid and Sodium Alginate.

7. The solid oral immediate-release pharmaceutical tablet formulation according to claim 6, wherein the disintegrant is Sodium starch glycolate.

8. The solid oral immediate-release pharmaceutical tablet formulation according to any one of claims 1 to 7, wherein the at least one binder is selected from Povidone, Hydroxypropyl Cellulose, Hydroxypropyl Methylcellulose, Polyvinyl Alcohol, Pregelatinized Starch, Sodium Alginate, Microcrystalline Cellulose + Silica, and combinations thereof.

9. The solid oral immediate-release pharmaceutical tablet formulation according to claim 8, wherein the binder is Povidone or Hydroxypropyl cellulose, or combination thereof.

10. The solid oral immediate-release pharmaceutical tablet formulation according to any one of claims 1 to 9, wherein the glidant is selected from Colloidal Silicon Dioxide, Calcium Phosphate Tribasic, Magnesium Stearate, and Talc, or combinations thereof.

11. The solid oral immediate-release pharmaceutical tablet formulation according to claim 10, wherein the glidant is Colloidal silicone dioxide.

12. The solid oral immediate-release pharmaceutical tablet formulation according to any one of claims 1 to 11, wherein the lubricant is selected from Magnesium Stearate, Calcium Stearate, Stearic Acid, Sodium Stearyl Fumarate, and Talc.

13. The solid oral immediate-release pharmaceutical tablet formulation according to claim 12, wherein the lubricant is Magnesium stearate.

14. The solid oral immediate-release pharmaceutical tablet formulation according to any one of claims 1 to 13, wherein the tablet comprises
from 3 % w/w to 6 % w/w of Ruxolitinib hemifumarate,
from 85 to 90 % w/w of a diluent,
from 2 to 6 % w/w of a disintegrant,
from 2 to 6 % w/w of a binder,
from 0.5 to 2 % w/w of a glidant, and
from 0.5 to 2 % w/w of a lubricant,
calculated based on the total weight of the tablet.

15. The solid oral immediate-release pharmaceutical tablet formulation according to claim 14, wherein the tablet comprises
3.7 % w/w of Ruxolitinib hemifumarate
87.8 % w/w of Microcrystalline Cellulose
3 % w/w of Sodium Starch Glycolate
4 % w/w of Povidone
1 % w/w of Colloidal Silicon Dioxide and
0.5 % w/w of Magnesium Stearate,
calculated based on the total weight of the tablet.

16. The solid oral immediate-release pharmaceutical tablet formulation according to claim 14, wherein the tablet comprises
3.7 % w/w of Ruxolitinib hemifumarate,
87.8 % w/w of Microcrystalline Cellulose,
3 % w/w of Sodium Starch Glycolate,
2 % w/w of Povidone,
2 % w/w of Hydroxypropyl cellulose,
1 % w/w of Colloidal Silicon Dioxide,
0.5 % w/w of Magnesium Stearate,
calculated based on the total weight of the tablet.

17. Method for preparation of the solid oral immediate-release pharmaceutical tablet formulation according to any one of claims 1 to 16, comprising a step of dry granulation of Ruxolitinib hemifumarate and the one or more pharmaceutically acceptable excipients selected from a diluent, a disintegrant, at least one binder, a glidant and a lubricant, to form a granulate; a step of combining the granulate with a disintegrant and a lubricant to form a mixture; and a step of direct compaction of the mixture; wherein all steps are performed solvent-free; and wherein the dry granulation preferably includes at least one of slugging, compaction and roller compaction.

18. Method for preparation of the solid oral immediate-release pharmaceutical tablet formulation according to any one of claims 1 to 16, comprising a step of combining Ruxolitinib hemifumarate and the one or more pharmaceutically acceptable excipients selected from a diluent, a disintegrant, at least one binder, a glidant and a lubricant, to form a mixture; and a step of direct compaction of the mixture; wherein all steps are performed solvent-free.
